# FASCICULE DE BREVET EUROPEEN

(11) **EP 0 888 131 B1**
(45) Date de publication et mention de la délivrance du brevet: **03.04.2002**
(21) Numéro de dépôt: 97914404.5
(22) Date de dépôt: 14.03.1997
(51) Int. Cl.: A61L 2/00, B01D 15/08, C07K 14/765, C07K 1/16

(54) **PROCEDE POUR ELIMINER LES AGENTS TRANSMISSIBLES NON CONVENTIONNELS D'UNE SOLUTION PROTEIQUE**
VERFAHREN ZUM ENTFERNEN VON UNKONVENTIONELLEN KRANKHEITSERREGERN AUS PROTEINLÖSUNGEN
METHOD FOR REMOVING UNCONVENTIONAL TRANSMISSIBLE AGENTS FROM A PROTEIN SOLUTION

(30) Priorité: 15.03.1996 FR 9603549; 09.07.1996 FR 9608548
(43) Date de publication de la demande: 07.01.1999
(73) Titulaire: Aventis Pasteur, 69367 Lyon Cedex 07 (FR); IMEDEX, F-69630 Chaponost (FR)
(72) Inventeur: GRANDGEORGE, Michel, F-69670 Vaugneray (FR); LABATUT, René, F-69230 Saint-Genis-Laval (FR); ROUZIOUX, Jean-Marc, F-69160 Tassin-la-Demi-Lune (FR); TAYOT, Jean-Louis, F-69890 La-Tour-de-Salvagny (FR); VERON, Jean-Luc, F-67540 Ostwald (FR)
(74) Mandataire: Kerneis, Danièle
(86) Numéro de dépôt international: FR9700465
(87) Numéro de publication internationale: WO9734642

(56) Documents cités:
- EP-A- 0 307 373
- EP-A- 0 402 205
- EP-A- 0 530 173
- EP-A- 0 667 352
- WO-A-93/21190
- WO-A-94/24554
- DE-A- 4 429 558
- DEVELOPMENTS IN BIOLOGICAL STANDARDIZATION, vol. 88, 1996, BASEL CH, pages 257-264, XP000197507 R.W. KOZAK ET AL: "Transmissible spongiform encephalopathies (TSE): minimizing the risk of transmission by biological/pharmaceutical products: an industry perspective"
- CHEMICAL ABSTRACTS, vol. 121, no. 13, 26 Septembre 1994 Columbus, Ohio, US; abstract no. 155840q, K. IIDA ET AL.: "A new, validated method for the destruction of conventional viruses and unconventional infectious agents , (e.g. BSE and scrapie) in proteins and sera" page 884; colonne 1; XP002020258 & ANIM. CELL. TECHNOL. : BASIC APPL. ASPECTS, 1992, pages 295-300,

## Description

L'invention est relative au domaine de l'élimination des agents transmissibles non conventionnels (ATNC). Plus particulièrement, l'invention a trait à un procédé pour éliminer les ATNC d'une solution protéique.

Les agents transmissibles non conventionnels (ATNC), parmi lesquels les prions sont à l'origine des encéphalopathies subaiguës spongiformes transmissibles qui sont des maladies lentes dégénératives du système nerveux central, dont l'évolution est toujours fatale et dont les agents responsables ne sont pas toujours identifiés.
Ces encéphalopathies subaiguës spongiformes transmissibles concernent aussi bien l'homme que l'animal :
→ chez l'homme : le Kuru, la maladie de Creutzfelt-Jakob, le Syndrome de Gerstmann-Sträussler-Scheinker, l'insomnie fatale familiale et, pour certains, la maladie d'Alpers.
→ chez l'animal : la tremblante naturelle du mouton, l'encéphalopathie transmissible du vison, le syndrome du dépérissement chronique des ruminants sauvages en captivité et en liberté, et l'encéphalopathie spongiforme bovine.

L'élimination des ATNC dans les produits d'origine biologique pose des problèmes très difficiles et aujourd'hui très mal résolus, à quelques exceptions près.

Les méthodes d'inactivation aujourd'hui identifiées, telles que le traitement par la soude, par l'eau de Javel ou le chauffage à des températures supérieures à 130°C, sont inapplicables à la plupart des substances biologiques qui sont trop fragiles pour supporter de telles conditions physico-chimiques.

Les méthodes de séparation en vue d'éliminer les ATNC peuvent être théoriquement les mêmes que celles appliquées pour les protéines en général, c'est-à-dire la filtration. l'adsorption sélective, la chromatographie, l'ultracentrifugation, mais elles se heurtent à une très grande difficulté. Ces méthodes aujourd'hui ne sont pas appliquées quand il s'agit d'éliminer des virus traditionnels, car il reste toujours une partie de l'infectivité, ne serait-ce que minime, mais suffisante pour transmettre la maladie après injection du produit. On leur préfère, à juste titre, les méthodes d'inactivation. Le caractère transmissible des prions est aussi puissant que celui de la plupart des virus et on ne dispose pas aujourd'hui de méthodes de chromatographie connues pour éliminer avec certitude les dernières traces pouvant contaminer une solution protéique complexe. Des méthodes de filtration ont été proposées dans ce but et ont montré leur efficacité quand il s'agit de débarrasser des solutions de protéines de poids moléculaire inférieur à 100 kilodaltons. Au-delà, ces méthodes de tamisage se heurtent à la difficulté suivante : la taille des pores choisie pour arrêter les prions, arrête aussi les grosses protéines, soit parce que la protéine a un diamètre moyen hydraté supérieur à celui du pore, soit parce que des phénomènes d'adsorption compliquent la filtration de la protéine. Ces problèmes sont rencontrés en particulier dans la purification des sérums d'animaux utilisés dans les méthodes de culture cellulaire en vue de la préparation de vaccins ou de médicaments.

De même, lorsqu'on extrait de l'albumine d'un matériel biologique, qu'il soit humain ou animal, et que cette albumine est destinée à un usage thérapeutique, il est indispensable de la traiter afin d'en éliminer les agents transmissibles non conventionnels qui y seraient éventuellement présents.

On connaît par l'article "Viral Validation of the manufacturing process of high purity albumin from placentas" publié dans Brown F (ed) : Virological Safety Aspects of Plasma Derivatives Dev. Bio Stand. Basel, Karger, 1993, vol 81, pp. 237-244, un procédé de purification d'albumine à partir de placentas humains, présentant toutes les garanties de sécurité vis-à-vis d'éventuelles contaminations virales. Ainsi que cela a été mentionné dans l'article intitulé "Safety of placental blood derivatives" publié au Lancet, Vol 343. January 15, 1994, ce procédé a été considéré particulièrement adapté à l'élimination des prions. En effet, les étapes de purification réalisées dans ce procédé comportent notamment trois étapes FC1, FC2 et Hcol de précipitation alcoolique en milieu acide associées à des filtrations. Les prions ayant tendance à coprécipiter avec les protéines dénaturées, ces trois étapes, qui sont caractérisées par l'élimination de quantités importantes de précipités protéiques, sont particulièrement appropriées pour l'élimination des prions.

Cependant, ce procédé, s'il est bien adapté à la purification d'albumine placentaire, ne convient pas du tout à la purification de toutes les solutions protéiques et notamment pas à des solutions d'albuminc d'origine plasmatique. L'étape de précipitation FC1, par exemple, est une étape dont les conditions de réalisation sont telles que l'hémoglobine elle-même est dénaturée et précipitée de manière spécifique ; dans ie cas du plasma, la quantité d'hemogiobine est très faible ; cette étape n'aurait donc pas les mêmes caractéristiques et notamment l'albumine elle-même serait exposée à une dénaturation par l'agent chimique.

Il est donc souhaitable de pouvoir disposer d'un procédé pour éliminer les agents transmissibles non conventionnels d'une solution protéique, sans altérer de façon notable, les propriétés des protéines présentes dans la solution.

L'invention a pour but de proposer un tel procédé adapté au traitement d'une solution protéique aqueuse, qu'il s'agisse d'une solution purifiée ou non, et notamment d'une solution d'albumine, d'un sérum animal destiné à être utilisé pour effectuer de la culture cellulaire, d'une préparation d'immunoglobulines d'origine sérique ou d'une préparation de facteurs intervenant dans la coagulation.

Un autre but de l'invention est de proposer un procédé susceptible d'être utilisé de façon industrielle.

Pour atteindre ces buts, la présente invention a pour objet un procédé pour éliminer les agents transmissibles non conventionnels d'une solution protéique aqueuse, caractérisé en ce qu'il consiste essentiellement à traiter la solution protéique par chromatographie d'adsorption électrostatique et hydrophobe simultanées et à recueillir le filtrat.

Selon un mode de réalisation de l'invention, la solution protéique est traitée par chromatographie électrostatique et hydrophobe en deux étapes, une étape étant effectuée à pH acide, l'autre étape étant effectuée à pH sensiblement neutre.

Selon une caractéristique particulière, l'étape à pH acide est réalisée avant l'étape à pH sensiblement neutre.

Selon un autre mode de réalisation de l'invention, la solution protéique est traitée en outre par chromatographie échangeuse d'anions.

Selon une autre caractéristique, le procédé selon l'invention consiste en outre à filtrer au préalable la solution protéique au moyen d'un filtre dont le seuil de coupure est sensiblement égal à 0,2 µm. Ainsi, les colonnes de chromatographie sont protégées de tout colmatage ou contamination bactérienne.

La présente invention sera mieux comprise à la lecture de la description détaillée qui va suivre.

La solution protéique pour laquelle il convient d'éliminer les ATNC éventuellement présents peut avoir des origines diverses. Il peut s'agir de solutions protéiques non purifiées susceptibles d'être contaminées par des prions ou de solutions purifiées, d'origine sérique ou non, telles que les préparations d'immunoglobulines d'origine sérique et les préparations contenant des facteurs de coagulation. De façon particulière, il peut s'agir d'une solution d'albumine d'origine plasmatique obtenue par une méthode incluant la technique de fractionnement de Cohn. Dans tous les cas, la solution protéique à traiter est une solution aqueuse.

Le gel utilisé pour réaliser la chromatographie d'adsorption électrostatique et hydrophobe selon l'invention est un gel macroporeux qui possède des groupements fonctionnels, tels que des réticulats de diethylaminopolystyrène, de diéthylaminopolyphenyi ou toute autre association de radicaux porteurs d'une charge positive et de molécules hydrophobes, liés à une matrice inerte (par exemple silice, cellulose, sepharose, acrylamide). De préférence, on utilise du DEA-Spherosil/LS® fourni par la Société BIOSEPRA (USA, dont le siège français est situé 35 avenue Jean Jaurès - 92390 VILLENEUVE LA GARENNE).

Le support de la colonne peut être équilibré par des tampons classiques, par exemple des tampons phosphates de sodium, de préférence additionnés de chlorure de sodium.

Dans le cas particulier où cette chromatographie est effectuée en 2 étapes, il est possible d'utiliser pour chacune des étapes le même support, seul le pH changeant.

Selon un mode particulier de réalisation du procédé selon l'invention, la solution protéique est en outre traitée par chromatographie échangeuse d'anions, notamment par passage dans une colonne chargée en groupements diethylaminoethyl greffés sur une matrice inerte (par exemple dextran-silice, cellulose, sepharose, acrylamide) ; on utilise, par exemple, une colonne de DEAE-Spherodex/LS® fourni par la Société BIOSEPRA (déjà identifiée ci-dessus), que l'on équilibre au préalable par un tampon de manière à ce que la charge électrique en surface soit positive. On peut, par exemple, utiliser un tampon phosphate monosodique de pH 6,8.

Suivant la nature et l'usage attendu de la solution protéique traitée, il est possible d'adjoindre en outre, au procédé selon l'invention, par sécurité, une étape supplémentaire de chromatographie d'adsorption non spécifique au moyen par exemple d'une colonne de silice. On peut ainsi éliminer les éléments éventuellement relargués lors des étapes précédentes.

Selon un mode particulier du procédé selon l'invention, il est possible, après chaque traitement, de laver les colonnes de chromatographie grâce par exemple à des solutions de HCl ou à un mélange éthanol/HCl, ainsi que de procéder à un lavage particulier par une solution de NaOH, selon une fréquence librement choisie, tous les 5 cycles par exemple.

Lorsque les colonnes ont été lavées, elles peuvent être stockées, dans une solution d'éthanol à 70 % par exemple.

Le fait de pouvoir utiliser plusieurs fois les mêmes colonnes permet la mise en oeuvre du procédé selon l'invention au niveau industriel.

De façon surprenante, on a remarqué que, bien que les protéines et notamment l'albumine aient, comme les ATNC, tendance à s'adsorber sur un support hydrophobe, on pouvait grâce au procédé selon l'invention, séparer les ATNC d'une solution aqueuse de protéines et notamment d'albumine.

Les exemples qui suivent illustrent, de façon non limitative, quelques modes de réalisation de l'invention.

### Exemple 1 - Préparation d'un échantillon contaminé de sérum animal

En vue de démontrer les qualités du procédé selon l'invention, il est nécessaire de réaliser un échantillon contaminé de sérum animal (sérum de veau dans les exemples choisis, mais ceci est applicable de la même manière à d'autres espèces ou à d'autres solutions protéiques). La source de prions utilisée est un broyat de cerveaux de souris mâles C 57 BL/6 infectées par l'agent de la tremblante expérimentale de la souris (souche C 506/M3 au 7ème passage fournie par les Docteurs D.C. GAJDUSEK et P. BROWN du NIH à Bethesda, USA, dont le titre infectieux est environ 10⁸ LD50 par gramme de cerveau) et arrivées au stade terminal de la maladie :
1 ml de broyat de cerveau est soniqué et mélangé avec 20 ml de sérum.

### Exemple 2 - Premier mode de traitement de l'échantillon de sérum de veau contaminé

On prépare une colonne de DEA-Spherosil/LS® contenant 50 ml de support que l'on équilibre par un tampon de phosphate de sodium 0,02 M à pH 6,8 additionné de NaCl à hauteur de 2 g/l.
10 ml de sérum de veau, additionné de NaCl (2 g/l de sérum) et contaminé par la souche Scrapie selon l'exemple 1, sont ajustés à pH 6,8, filtrés sur membrane 0,2 µm et injectés sur la colonne de chromatographie. Le débit est ajusté à 100 ml/h pour une section de colonne égale à 5 cm², soit une vitesse linéaire de 20 cm/h.
Le filtrat, correspondant au pic d'adsorption UV à 280 nm, est collecté et filtré stérilement sur membrane de porosité 0,2 µm; des échantillons sont prélevés et stockés à - 70° C pour contrôles biologiques.

Après traitement, la colonne est lavée par passage de cinq volumes des solutions suivantes :
1/ HCl 0,1 N
2/ Ethanol 70 % - HCI 0,1 N
3/ NaOH 0,1 N

Le stockage de la colonne est assuré en éthanol 70 %.

### Exemple 3 - Second mode de traitement de l'échantillon de sérum de veau contaminé

Dans ce cas, on prépare en outre une colonne de DEAE-Spherodex/LS® contenant 50 ml de support que l'on équilibre également par un tampon phosphate de sodium 0,02 M à pH 6.8 additionné de NaCl pour obtenir une concentration de 2 g/l.

Cette colonne est placée en série avant la colonne de DEA-Spherosil/LS® de l'exemple 2.

La solution à traiter est la même qu'à l'exemple 2.

On procède dans les mêmes conditions qu'à l'exemple 2 à l'exception du fait que la solution est traitée successivement par chromatographie échangeuse d'anions grâce à la colonne de DEAE-Spherodex/LS® puis par chromatographie d'adsorption électrostatique et hydrophobe grâce à la colonne de DEA-Spherosil/LS®.

Les échantillons prélevés sont également stockés à -70°C pour contrôles biologiques.

### Exemple 4 - Vérification de l'absence de contamination de la colonne

Après un premier cycle de purification conforme à l'exemple 2 ou à l'exemple 3, et après 15 h de stockage des colonnes en éthanol 70 %, un nouveau cycle de purification est réalisé avec 10 ml de sérum de veau non contaminé. Le filtrat est également récolté et des échantillons sont prélevés pour titrages biologiques. Ces échantillons sont analysés pour montrer l'absence de particules transmissibles de prions pouvant venir du cycle de purification précédent.

### Exemple 5- Troisième mode de traitement de l'échantillon de sérum de veau contaminé

La même expérience que celle décrite à l'exemple 3 est réalisée à pH 5,0 au lieu de 6,8.

Les deux colonnes de DEAE-Spherodex/LS® et de DEA-Spherosil/LS® sont équilibrées par un tampon phosphate de sodium 0,02 M à pH 5,0 additionné de NaCl à hauteur de 2 g/l.
Le sérum est additionné de NaCl 2 g/l, ajusté lui aussi à pH 5,0 par addition d'HCl et filtré sur membrane de porosité 0,2 µm.
La suite de l'expérience est identique à l'exemple 3.
En fin de purification, le sérum récolté est ajusté à pH neutre par addition de NaOH N, avant d'être échantillonné et stocké pour contrôles biologiques.

### Exemple 6 - Quatrième mode de traitement de l'échantillon de sérum de veau contaminé

Dans ce cas, le passage sur DEAE-Sphérodex/LS® se fait dans un premier temps et on réalise le passage sur DEA-Spherosil/LS® dans un deuxième temps.

Le passage sur DEAE-Spherodex/LS® se fait à pH 6,8 après filtration 0,2 µm, selon les conditions de l'exemple 3 (sans la colonne DEA-Spherosil/LS®).

La fraction récoltée est alors ajustée à pH 5,0 et injectée sur la colonne DEA-Spherosil/LS® dans les conditions de l'exemple 5 (sans la colonne DEAE-Spherodex/LS®).

En fin de purification, le sérum récolté est ajusté à pH neutre par addition de NaOH N, avant d'être échantillonné et stocké pour contrôles biologiques.

### Exemple 7 - Cinquième mode de traitement de l'échantillon du sérum de veau contaminé

Cet exemple est une variante de l'exemple 6, dans lequel les conditions de passage sur la première colonne de DEAE-Spherodex/LS® sont différentes.

Le sérum contaminé est dialysé contre du tampon phosphate sodique 0,02 M, pH 6,8 (sans addition de NaCl). La même quantité de sérum ainsi traitée est filtrée sur membrane de porosité 0,2 µm et injectée sur une colonne identique de DEAE-Spherodex/LS® équilibrée en phosphate sodique 0,02 M, pH 6,8 (sans addition de NaCl).

Dans ces conditions, une partie importante des protéines se fixe sur le support, une autre partie ne se fixe pas et est récoltée dans une première fraction (filtrat).

La fraction fixée est éluée par injection de tampon additionné de NaCl à hauteur de 10 g/l. La deuxième fraction correspondant à l'éluat est récoltée. L'ensemble des deux fractions (filtrat et éluat) est mélangé pour être filtré sur membrane de porosité 0,2 µm et appliqué sur la colonne de DEA-Spherosil/LS® équilibrée à pH 6,8, comme dans l'exemple 2.

Le filtrat final récolté est échantillonné et stocké pour contrôles biologiques.

### Exemple 8 - Sixième mode de traitement de l'èchantillon de sérum de veau contaminé

Cet exemple est semblable à l'exemple 7, sauf que le mélange recueilli à la sortie de la colonne DEAE-Spherodex/LS® est ajusté à pH 5,0 et que la chromatographie sur la deuxiéme colonne de DEAE-Spherosil/LS® est effectuée en tampon phosphate 0,02 M, pH 5,0 additionné de NaCl à hauteur de 5 g/l.

### Exemple 9 - Contrôles biologiques des échantillons de sérum de veau traité

Les échantillons prélevés à la suite de chacun des traitements effectués selon les exemples 2 à 8 et stockés à - 70° C, sont contrôlés grâce à des tests biologiques classiques effectués sur souris et montrent que :
- le sérum animal ainsi traité ne contient plus d'éléments capables de transmettre la tremblante aux souris,
- le sérum animal ainsi traité conserve ses propriétés de stimulation de la multiplication cellulaire et peut être utilisé à la place du sérum animal disponible aujourd'hui.

### Exemple 10 - Traitement d'une première solution d'albumine

On dispose de 150 ml d'une solution d'albumine d'origine placentaire purifiée partiellement selon les 4 étapes de précipitation et l'étape de dilution décrites p: 239 de l'article "Viral Validation of the Manufacturing Process of High Purity Albumin From Placentas" mentionné plus haut.

Cette solution est une solution hydroéthanolique d'albumine à 50 g/l d'albumine.

On contamine volontairement cette solution par 2 % (v/v) d'un inoculum contenant des ATNC. Cet inoculum est obtenu par broyage de cerveaux d'animaux (souris C57BL/6 mâles) infectés par l'agent de la tremblante expérimentale de la souris (souche C 506/M3 au 7ème passage fourni par les Drs. D.C. Gajdusek et P. Brown, N.I.H. Bethesda USA dont le titre infectieux est environ 10⁸ LD50 par gramme de cerveau) et arrivés au stade terminal de la maladie. Le broyat est effectué dans une solution stérile de glucose à 5 %, selon un rapport poids/volume de 10 %. L'inoculum est soniqué, puis clarifié par centrifugation et filtré sur filtre 0,45 µm avant utilisation.

La solution d'albumine contaminée est filtrée sur filtre 0,2 µm puis traitée successivement par chromatographie DEAE-Spherodex/LS® , puis DEA-Spherosil/LS® à pH 4,8 et enfin DEA-Spherosil/LS® à pH 6,8.

La 1ère colonne ou DEAE-Spherodex/LS® contenant 30 g de gel est tout d'abord équilibrée par du tampon phosphate monosodique à pH 6,8.

On injecte ensuite la solution d'albumine dans la colonne. L'albumine, chargée négativement se fixe progressivement sur le support par interaction ionique. L'alcool et les impuretés protéiques qui sont électriquement neutres ou qui sont chargées positivement sont directement éliminées.
La colonne est ensuite rincée par une solution alcoolique à 12,5 % puis par de l'eau purifiée.
L'albumine est ensuite éluée de la colonne grâce au passage d'une solution à 10 g/l de NaCl. La colonne est ensuite régénérée au moyen d'une solution à 60 g/l de NaCl puis stérilisée grâce à une phase de régénération acide par lavage au HCl 0,1 N ; la colonne est ensuite rincée dans une solution alcoolique à 70 % et conservée dans cette solution.

La solution saline d'albumine obtenue est alors soumise à une chromatographie sur colonne DEA Spherosil/LS® contenant 15 g de gel à pH 4,8. Cette chromatographie combine l'adsorption électrostatique grâce aux groupements diethylamino et l'adsorption hydrophobe grâce aux réticulats de polystyrène. Le support de la colonne est au préalable équilibré par une solution de NaCl à 0,7 g/l. Lors du passage de la solution d'albumine dans cette colonne, certaines impuretés hydrophobes sont fixées alors que l'albumine reste en solution.
La colonne est ensuite rincée par passage d'une solution NaCl à 4 g/l.
La colonne est ensuite régénérée par lavage avec de l'HCl 0,1 N, puis lavée par une solution alcoolique à 70 % et stockée dans l'alcool.

La solution d'albumine obtenue à la sortie de cette colonne est ensuite soumise à une nouvelle étape de chromatographie sur un support identique, mais cette fois à pH 6,8. La colonne contient ici 3 g de gel et est associée à une colonne contenant 3 g de silice. Cette étape permet d'adsorber les impuretés hydrophobes, non fixées au pH auquel est réalisée l'étape précédenté ; elle permet également d'éliminer d'éventuelles molécules de DEAE - dextran provenant de la 1ère chromatographie.
Les 2 colonnes sont ensuite soumises à un premier rinçage par une solution de NaCl à 6 g/l afin de récupérer l'albumine éventuellement retenue.
Puis les colonnes sont soumises à un second rinçage avec une solution concentrée de NaCl à 60 g/l afin d'éliminer les traces éventuelles d'albumine dénaturée.
On régénère ensuite les colonnes par lavage avec de l'HCl 0,1 N puis on les stérilise par lavage avec une solution alcoolique à 70 % et on les conserve dans de l'alcool.
On obtient à la sortie de la colonne de silice une solution d'albumine pure concentrée ensuite à 7,6 ml.

Afin de vérifier la présence d'ATNC, on effectue un test sur environ 200 souris saines C 57BL/6 mâles de 18-20 grammes à chacune desquelles on inocule 20 µl de solution d'albumine par voie intracérébrale au niveau de l'hémisphère droit. Les 24 premières souris reçoivent de la solution d'albumine concentrée. Ensuite, la solution d'albumine est diluée de 10 en 10 avant d'être injectée, chacune des dilutions étant injectée à 12 souris.
On maintient les animaux sous observation afin de vérifier les signes d'apparition de la tremblante expérimentale de la souris. Une souris est considérée positive après la seconde évaluation positive d'un signe clinique. Lorsqu'une souris meurt, on congèle son cerveau à - 80°C et on effectue un examen histopathologique afin de confirmer l'origine de la mort.
Les résultats obtenus montrent que, grâce au procédé selon l'invention, la réduction du titre infectieux de la solution d'albumine du départ exprimé en DL50 (qui est déterminée par la méthode de Reed et Muench pour des dilutions réalisées de 10 en 10 de l'échantillon testé), est de 10^{5,5}, soit une différence de titre de 5,5 log.

### Exemple 11 - Vérification de l'absence de contamination des colonnes

Après avoir réalisé l'exemple 10, on dispose de 150 ml d'une solution d'albumine à 50 g/l identique à celle de l'exemple 10, exception faite de la contamination volontaire par un inoculum contenant des ATNC ; on fait passer cette solution d'albumine par les mêmes étapes de chromatographie que celles décrites à l'exemple 10, afin de vérifier que les ATNC adsorbés par les colonnes de chromatographie lors de l'exemple 10, ne sont pas relargués dans la nouvelle solution d'albumine. On obtient cette fois 6,6 ml de solution purifiée.
On effectue donc le même test qu'à l'exemple 10, c'est-à-dire inoculation intracérébrale, à chaque animal, de 20 µl de la solution d'albumine ainsi obtenue, puis de la solution d'albumine diluée de 10 en 10. Environ 100 souris sont ainsi inoculées.
Les résultats obtenus montrent que les souris ne sont pas contaminées ; la solution d'albumine est donc indemne de tout ATNC.
Il est donc possible d'utiliser les colonnes de chromatographie à plusieurs reprises.

### Exemple 12 - Traitement d'une deuxième solution d'albumine

On réalise la même expérience qu'à l'exemple 10 en partant cette fois d'une solution d'albumine constituée par la fraction V de la technique de COHN et filtrée sur filtre 0,45 µm.
La solution d'albumine alors obtenue présente les mêmes caractéristiques que celle obtenue à l'exemple 10.

## Revendications

1. Procédé pour éliminer les agents transmissibles non conventionnels (ATNC) d'une solution protéique aqueuse, **caractérisé en ce qu'**il consiste essentiellement à traiter la solution protéique par chromatographie d'adsorption électrostatique et hydrophobe simultanées et à recueillir le filtrat.

2. Procédé selon la revendication 1, **caractérisé en ce que** la chromatographie d'adsorption électrostatique et hydrophobe simultanées est réalisée en deux étapes, une étape étant effectuée à pH acide, l'autre étape étant effectuée à pH sensiblement neutre.

3. Procédé selon la revendication 2, **caractérisé en ce que** l'étape à pH acide est réalisée avant l'étape à pH sensiblement neutre.

4. Procédé selon une des revendications précédentes, **caractérisé en ce qu'**il consiste en outre à traiter la solution protéique aqueuse par chromatographie échangeuse d'anions.

5. Procédé selon une des revendications précédentes **caractérisé en ce que** la chromatographie d'adsorption électrostatique et hydrophobe est réalisée au moyen d'un gel possédant des groupements fonctionnels, tels que des réticulats de diéthylaminopolystyrène, de diéthylaminopolyphényl ou toute autre association de radicaux porteurs d'une charge positive et de molécules hydrophobes, liés à une matrice inerte.

6. Procédé selon une des revendications précédentes, **caractérisé en ce que** la chromatographie d'adsorption électrostatique et hydrophobe est effectuée au moyen d'un support macroporeux possédant des groupements diéthylaminés.

7. Procédé selon une des revendications précédentes, **caractérisé en ce que** la chromatographie d'adsorption électrostatique et hydrophobe est réalisée au moyen du gel DEA-Spherosil/LS®.

8. Procédé selon une des revendications précédentes, **caractérisé en ce qu'**il consiste en outre à filtrer au préalable la solution protéique, au moyen d'un filtre dont le seuil de coupure est sensiblement égal à 0,2 µm.

9. Procédé selon une des revendications précédentes, **caractérisé en ce qu'**il consiste à traiter une solution d'albumine.

10. Procédé selon une des revendications 1 à 8, **caractérisé en ce qu'**il consiste à traiter une solution comprenant du sérum de veau.

11. Procédé selon une des revendications 1 à 8, **caractérisé en ce qu'**il consiste à traiter une solution de protéines plasmatiques.

## Patentansprüche

1. Verfahren zur Entfernung von unkonventionellen transmissiblen Agenzien (UTAs) aus einer wäßrigen Proteinlösung, **dadurch gekennzeichnet, daß** man dabei im wesentlichen die Proteinlösung mittels gleichzeitiger elektrostatischer und hydrophober Adsorptionschromatographie behandelt und das Filtrat auffängt.

2. Verfahren nach Anspruch 1, **dadurch gekennzeichnet, daß** man die gleichzeitige elektrostatische und hydrophobe Adsorptionschromatographie in zwei Schritten durchführt, wobei man einen Schritt bei saurem pH und den anderen Schritt bei weitgehend neutralem pH vornimmt.

3. Verfahren nach Anspruch 2, **dadurch gekennzeichnet, daß** man den Schritt bei saurem pH vor dem Schritt bei weitgehend neutralem pH durchführt.

4. Verfahren nach einem der vorhergehenden Ansprüche, **dadurch gekennzeichnet, daß** man außerdem auch noch die wäßrige Proteinlösung mittels Anionenaustauschchromatographie behandelt.

5. Verfahren nach einem der vorhergehenden Ansprüche, **dadurch gekennzeichnet, daß** man die elektrostatische und hydrophobe Adsorptionschromatographie mit Hilfe eines Gels mit funktionellen Gruppen, wie vernetztem Diethylaminopolystyrol, Diethylaminopolyphenyl oder einer anderen Kombination von Resten mit positiver Ladung und hydrophoben Molekülen, die an eine inerte Matrix gebunden sind, durchführt.

6. Verfahren nach einem der vorhergehenden Ansprüche, **dadurch gekennzeichnet, daß** man die elektrostatische und hydrophobe Adsorptionschromatographie mit Hilfe eines makroporösen Trägers mit Diethylaminogruppen durchführt.

7. Verfahren nach einem der vorhergehenden Ansprüche, **dadurch gekennzeichnet, daß** man die elektrostatische und hydrophobe Adsorptionschromatographie mit Hilfe von DEA-Spherosil/LS®-Gel durchführt.

8. Verfahren nach einem der vorhergehenden Ansprüche, **dadurch gekennzeichnet, daß** man außerdem auch noch die Proteinlösung zunächst mit Hilfe eines Filters mit einer Trenngrenze von im wesentlichen 0,2 µm filtriert.

9. Verfahren nach einem der vorhergehenden Ansprüche, **dadurch gekennzeichnet, daß** man eine Albuminlösung behandelt.

10. Verfahren nach einem der Ansprüche 1 bis 8, **dadurch gekennzeichnet, daß** man eine Kälberserum enthaltende Lösung behandelt.

11. Verfahren nach einem der Ansprüche 1 bis 8, **dadurch gekennzeichnet, daß** man eine Plasmaproteine enthaltende Lösung behandelt.

## Claims

1. Process for removing unconventional transmissible agents (UCTA) from an aqueous protein solution, **characterized in that** it essentially consists in treating the protein solution by simultaneous electrostatic and hydrophobic adsorption chromatography and collecting the filtrate.

2. Process according to Claim 1, **characterized in that** the simultaneous electrostatic and hydrophobic adsorption chromatography is carried out in two steps, with one step being carried out at acid pH and the other step being carried out at approximately neutral pH.

3. Process according to Claim 2, **characterized in that** the step at acid pH is carried out before the step at approximately neutral pH.

4. Process according to one of the preceding claims, **characterized in that** it additionally consists in treating the aqueous protein solution by anion exchange chromatography.

5. Process according to one of the preceding claims, **characterized in that** the electrostatic and hydrophobic adsorption chromatography is carried out using a gel which possesses functional groups, such as crosslinked products of diethylaminopolystyrene or diethylaminopolyphenyl, or any other combination of positive charge-carrying radicals and hydrophobic molecules linked to an inert matrix.

6. Process according to one of the preceding claims, **characterized in that** the electrostatic and hydrophobic adsorption chromatography is carried out using a macroporous support which possesses diethylamino groups.

7. Process according to one of the preceding claims, **characterized in that** the electrostatic and hydrophobic adsorption chromatography is carried out using DEA-Spherosil/LS® gel.

8. Process according to one of the preceding claims, **characterized in that** it additionally consists in carrying out a preliminary filtration of the protein solution using a filter whose cut-off threshold is approximately equal to 0.2 µm.

9. Process according to one of the preceding claims, **characterized in that** it consists in treating a solution of albumin.

10. Process according to one of Claims 1 to 8, **characterized in that** it consists in treating a solution which comprises calf serum.

11. Process according to one of Claims 1 to 8, **characterized in that** it consists in treating a solution of plasma proteins.
